(19) Europäisches Patentamt
European Patent Office
Office européen des brevets

(11) Veröffentlichungsnummer: **0 273 008**
**A2**

(12) **EUROPÄISCHE PATENTANMELDUNG**

(21) Anmeldenummer: **87810751.5**

(22) Anmeldetag: **14.12.87**

(51) Int. Cl.[4]: **C 07 F 7/18**
C 07 C 69/65

(30) Priorität: **18.12.86 CH 5058/86**

(43) Veröffentlichungstag der Anmeldung:
**29.06.88 Patentblatt 88/26**

(84) Benannte Vertragsstaaten:
**BE CH DE FR GB IT LI NL**

(71) Anmelder: **CIBA-GEIGY AG**
**Klybeckstrasse 141**
**CH-4002 Basel (CH)**

(72) Erfinder: **Lang, Robert Werner, Dr.**
**Hagenbachweg 10**
**CH-4133 Pratteln (CH)**

(54) **Verfahren zur Herstellung von 2,2-Difluorpent-4-en-carbonsäuren und -säurederivaten.**

(57) Die Umsetzung von Brom- bzw. Chlordifluoressigsäureallylestern mit Monochlorsilanen in Gegenwart von Zink unter Erwärmen ergibt Silylester von α,α-Difluor-γ,δ-pentensäuren. Durch Hydrolyse erhält man die freien Säuren, aus denen nach üblichen Methoden Säurederivate herstellbar sind. Die Pentensäuren und Pentensäurederivate eignen sich zur Herstellung von Polymeren und Copolymeren, aus denen metallorganische Polymere und Copolymere mit katalytischen Eigenschaften erhältlich sind.

EP 0 273 008 A2

**Beschreibung**

Verfahren zur Herstellung von 2,2-Difluorpent-4-en-carbonsäuren und -säurederivaten

Die Erfindung betrifft ein Verfahren zur Herstellung von 2,2-Difluorpent-4-en-carbonsäuren und -carbonsäurederivaten durch Umlagerung von Brom- bzw. Chlordifluoressigsäureallylestern in Gegenwart von Zink und Monochlorsilanen, sowie substituierte 2,2-Difluorpent-4-en-carbonsäuren und -carbonsäurederivate und Chlordifluoressigsäureallylester.

J.F. Normant et al. beschreiben im Bulletin de la Société Chimique de France, No. 9-10, S. 2077 (1974), die Herstellung von 2,2-Di-fluorpent-4-en-1-carbonsäure durch die Umsetzung von Allylalkohol mit Tetrafluorethylen, gefolgt von einer Behandlung mit Lithiumbutyl und einer anschliessenden Hydrolyse. Diese Labormethode eignet sich nicht für ein Verfahren im technischen Massstab.

In Tetr. Lett., Vol. 27, No. 37, pp 4437-4440 (1986) werden neben der 2,2-Difluorpent-4-en-1-carbonsäure auch deren Säurechlorid und Säureanhydrid beschrieben.

Ein Gegenstand vorliegender Erfindung ist ein Verfahren zur Herstellung von Verbindungen der Formel I

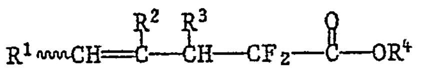

(I)

worin ~ für Cis-/Transisomere steht, $R^1$ H, $C_1$-$C_{24}$-Alkyl oder gegebenenfalls in der Arylgruppe mit $C_1$-$C_6$-Alkoxy, $C_1$-$C_6$-Alkylthio, F, Cl, Br oder -CN substituiertes $C_6$-$C_{12}$-Aryl, $C_7$-$C_{16}$-Aralkyl oder $C_8$-$C_{40}$-Alkaralkyl darstellt, $R^2$ und $R^3$ unabhängig voneinander H oder $C_1$-$C_{24}$-Alkyl bedueten, $R^1$ und $R^3$ zusammen gegebenenfalls mit $C_1$-$C_6$-Alkyl substituiertes $C_1$-$C_5$-Alkylen oder $C_2$-$C_5$-Alkenylen darstellen, $R^2$ und $R^3$ zusammen oder $R^1$ und $R^2$ zusammen gegebenenfalls mit $C_1$-$C_6$-Alkyl substituiertes $C_1$-$C_6$-Alkylen oder $C_3$-$C_6$-Alkenylen bedeuten, und $R^4$ für -$SiR^5R^6$uu$R^7$ steht, worin $R^5$, $R^6$ und $R^7$ unabhängig voneinander $C_1$-$C_{12}$-Alkyl, Phenyl oder Benzyl bedeuten, sowie deren Säuren und Säurederivate, das dadurch gekennzeichnet ist, dass man 1 Mol eines Monobrom- oder Monochlordifluoressigsäureallylesters der Formel II

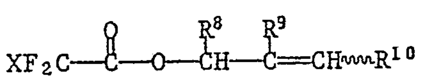

(II),

worin ~ die zuvor angegebene Bedeutung hat, X für Cl oder Br steht, $R^8$ die Bedeutungen von $R^1$, $R^9$ die Bedeutungen von $R^2$, $R^{10}$ die Bedeutungen von $R^3$, $R^8$ und $R^9$ die Bedeutungen von $R^1$ und $R^2$, $R^8$ und $R^{10}$ die Bedeutungen von $R^1$ und $R^3$ und $R^9$ und $R^{10}$ die Bedeutungen von $R^2$ und $R^3$ haben, in Gegenwart von mindestens 1 Mol Zink und von mindestens 1 Mol eines Monochlorsilans $R^5R^6R^7SiCl$, worin $R^5$, $R^6$ und $R^7$ die zuvor angegebene Bedeutung haben, erwärmt, die Verbindungen der Formel I isoliert oder zur Herstellung der Säuren hydrolisiert und gegebenenfalls aus den Säuren deren Säurederivate herstellt.

X steht bevorzugt für Cl.

$R^1$ in der Bedeutung von Alkyl kann verzweigtes und besonders lineares Alkyl mit 1 bis 24, bevorzugt 1 bis 20 und besonders 1 bis 12 C-Atomen sein. Beispiele sind: Methyl, Ethyl, n- und 1-Propyl, n-, i- und t-Butyl, 2-Methylbut-4-yl, n-Pentyl, 3-Methylpent-1-yl, Pent-2-yl, Pent-3-yl, n-Hexyl, Hex-2-yl, Hex-3-yl, n-Heptyl, Hept-2-yl, Hept-3-yl, 2-Ethylhex-1-yl, n-Octyl, Oct-2-yl, Oct-3-yl, Oct-4-yl, Nonyl, Decyl, Undecyl, Dodecyl, Tridecyl, Tetradecyl, Pentadecyl, Hexadecyl, Heptadecyl, Octadecyl, Nonadecyl und Eicosyl.

$R^1$ kann in der Bedeutung von Aryl, Aralkyl oder Alkaralkyl mit 1 bis 3, vorzugsweise 1 oder 2, $C_1$-$C_6$-, bevorzugt $C_1$-$C_4$-Aloxygruppen oder -Alkylthiogruppen oder F, Cl, Br oder -CN substituiert sein. Beispiele für Alkoxy und Alkylthio sind Methoxy, Ethoxy, n-und i-Propoxy, n-, i- und t-Butoxy, Pentoxy, Hexoxy, Methylthio, Ethylthio, Propylthio und Butylthio.

$R^1$ enthält als Aryl bevorzugt 6 bis 10 C-Atome und ist besonders Phenyl oder Naphthyl. $R^1$ als Aralkyl enthält bevorzugt 7 bis 12 C-Atome und stellt bevorzugt Phenylalkyl mit 1 bis 3 C-Atomen in der Alkylengruppe dar. $R^1$ als Alkaralkyl enthält bevorzugt 8 bis 34 C-Atome und stellt bevorzugt Alkylphenylalkyl dar, das insbesondere 1 bis 12 C-Atome in der Alkylgruppe und 1 bis 3 C-Atome in der Alkylengruppe enthält. Beispiele sind: Phenyl, Benzyl, 1-Phenyleth-2-yl, 1-Phenylprop-1-yl, Methylphenyl, Dimethylphenyl, Ethylphenyl, Ethylmethylphenyl, Propylphenyl, Butylphenyl, Hexylphenyl, Decylphenyl, Methylbenzyl, Dimethylbenzyl, Ethylbenzyl, Propylbenzyl, Butylbenzyl, Octylbenzyl, Dodecylbenzyl, 1-(Methylphenyl)eth-1-yl, 1-(Dimethylphenyl)-prop-1-yl und 1-(Octylphenyl)eth-1-yl.

$R^2$ und $R^3$ können unabhängig voneinander verzweigtes und bevorzugt lineares $C_1$-$C_{24}$-, bevorzugt $C_1$-$C_{12}$- und besonders $C_1$-$C_6$-Alkyl sein. Beispiele für Alkyl sind für die Gruppe $R^1$ zuvor genannt worden. Bevorzugt stellen $R^2$ und $R^3$ je H dar.

$R^1$ und $R^3$, $R^1$ und $R^2$ und $R^3$ zusammen in der Bedeutung von Alkylen oder Alkenylen können mit 1 bis 3, vorzugsweise 1 oder 2 Alkylgruppen mit 1 bis 6, bevorzugt 1 bis 4 C-Atomen substituiert sein. Beispiele sind:

Methyl, Ethyl, n- und i-Propyl, n-, i- und t-Butyl, Pentyl und Hexyl.

$R^1$ und $R^3$ zusammen in der Bedeutung von Alkylen enthalten bevorzugt 1 bis 3 C-Atome. $R^1$ und $R^3$ zusammen in der Bedeutung von Alkenylen enthalten bevorzugt 2 oder 3 C-Atome. $R^2$ und $R^3$ zusammen und $R^1$ und $R^2$ zusammen in der Bedeutung von Alkylen enthalten bevorzugt 1 bis 4 C-Atome. $R^2$ und $R^3$ zusammen in der Bedeutung von Alkenylen enthalten bevorzugt 3 oder 4 C-Atome. $R^1$ und $R^2$ zusammen in der Bedeutung von Alkenylen enthalten bevorzugt 3 oder 4 C-Atome. Beispiele für Alkylen sind Methylen, Ethylen, n-Propylen, n-Butylen, n-Pentylen und n-Hexylen. Beispiele für Alkenylen sind Ethenylen, n-Propenylen, n-But-1-enylen, n-But-2-enylen, n-Pentenylen und n-Hexenylen.

In den Verbindungen der Formel I stellt $R^4$ eine $R^5R^6R^7Si$-Gruppe dar, worin $R^5$, $R^6$ und $R^7$ als Alkyl bevorzugt lineares oder verzweigtes $C_1$-$C_8$-, besonders $C_1$-$C_6$- und insbesondere $C_1$-$C_4$-Alkyl darstellen. Besonders bevorzugt sind $R^5$, $R^6$ und $R^7$ gleiche oder verschiedene Alkylreste mit insbesondere 1 bis 8 C-Atomen.

Beispiele für $R^5$, $R^6$ und $R^7$ sind: Methyl, Ethyl, n-Propyl, i-Propyl, n-, i- oder t-Butyl, Pent-1-yl, 1-, 2- oder 3-Hexyl, 1,2,2-Trimethyleth-1-yl, 1,1,2,2-Tetramethyleth-1-yl (Thexyl), Heptyl, Octyl, Decyl, Dodecyl, Phenyl und Benzyl. Bevorzugt sind $R^5$, $R^6$ und $R^7$ Methyl.

Beispiele für die $R^5R^6R^7Si$-Gruppe sind: Methyldiethyl-, Dimethyl-pentyl-, Trimethyl-, Ethyldipropyl-, Methylethylpropyl-, Diethylpropyl-, Triethyl-, Dimethylethyl-, n-Propyldibutyl-, i-Propyldiethyl-, i-Propyldimethyl-, Tri-n-Propyl-, Tri-n-Butyl-, n-Butyldimethyl-, n-Butyldiethyl-, t-Butyldimethyl-, Tri-n-Pentyl-, n-Pentyldimethyl-, (1,2,2-Trimethyleth-1-yl)dimethyl-, (1,1,2,2-Tetramethylethyl)dimethyl-, Tri-n-Octyl- und n-Octyldimethylsilyl.

Eine bevorzugte Ausführungsform der erfindungsgemässen Verfahrens ist dadurch gekennzeichnet, dass $R^1$ H, $C_1$-$C_{20}$-Alkyl, gegebenenfalls in der Phenylgruppe mit $C_1$-$C_6$-Alkoxy, $C_1$-$C_6$-Alkylthio, F, Cl, Br oder -CN substituiertes Phenyl, $C_7$-$C_{16}$-Phenylalkyl oder $C_8$-$C_{30}$-Alkylphenylalkyl bedeutet, $R^2$ und $R^3$ unabhängig voneinander H oder $C_1$-$C_{12}$-Alkyl, $R^1$ und $R_3$ zusammen $C_1$-$C_3$-Alkylen oder $C_2$-$C_3$-Alkenylen darstellen, $R^2$ und $R^3$ zusammen oder $R^1$ und $R^2$ zusammen $C_1$-$C_4$-Alkylen oder $C_3$-$C_4$-Alkenylen sind.

In einer weiteren bevorzugten Ausführungsform des Verfahrens stehen $R^1$ für H oder $C_1$-$C_{20}$-Alkyl und $R^2$ und $R^3$ für H oder $C_1$-$C_6$-Alkyl, besonders für H.

Mit dem erfindungsgemässen Verfahren können auch Säuren und Säurederivate hergestellt werden. Im Falle der Säuren steht in Formel I $R^4$ für H. Bei den Säurederivaten kann es sich um Anhydride, Ester, Amide, Halogenide oder Salze der Carbonsäuren der Formel I, worin $R^4$ H ist, handeln.

Eine bevorzugte Gruppe von Säuren und Säurederivaten sind solche, worin $R^4$ in Formel I H, $C_1$-$C_{18}$-Acyl, der um eine Hydroxylgruppe verminderte Rest eines einwertigen Alkohols, ein Metall- oder Ammoniumkation, oder die Gruppe $R^4O$- für F, Cl, Br, $-NH_2$ oder den um ein H-Atom verminderten Rest eines primären oder sekundären Amins steht.

Im Fall der Anhydride entspricht der Rest $R^4$ z.B. der Formel

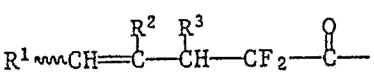 ,

worin ∿, $R^1$, $R^2$ und $R^3$ die zuvor angegebene Bedeutung haben. Im Falle von gemischten Anhydriden steht $R^4$ für einen $C_1$-$C_{18}$-, vorzugsweise $C_1$-$C_{12}$-Acylrest. Der Acylrest kann der Formel $R^{11}CO$- entsprechen, worin $R^{11}$ H oder gegebenenfalls mit Halogen, besonders F oder Cl substituiertes lineares oder verzweigtes $C_1$-$C_{12}$-, besonders $C_1$-$C_6$-Alkyl, Cyclopentyl, Cyclohexyl, Phenyl, Benzyl oder Phenylethyl ist. Beispiele für $R^{11}$ sind Methyl, Ethyl, n- und i-Propyl, n-, i- und t-Butyl, Pentyl, Hexyl, Chlormethyl, Dichlormethyl, Trichlormethyl, Fluormethyl, Difluormethyl, Trifluormethyl, Chlordifluormethyl und Dichlorfluormethyl.

Im Falle der Ester bedeutet $R^4$ in Formel I den um eine Hydroxylgruppe verminderten Rest eines Alkohols mit vorzugsweise 1 bis 20, besonders 1-12 und insbesondere 1 bis 8 C-Atomen. $R^4$ kann z.B. lineares oder verzweigtes $C_1$-$C_{20}$-besonders $C_1$-$C_{12}$-und insbesondere $C_1$-$C_6$-Alkyl, $C_5$- oder $C_6$-Cycloalkyl, Phenyl, $C_1$-$C_6$-Alkylphenyl, Phenyl-$C_1$-$C_3$-alkyl oder $C_1$-$C_6$-Alkylphenyl-$C_1$-$C_3$alkyl sein. Beispiele für solche Reste sind zuvor für $R^1$ angegeben worden.

Im Falle der Amide entspricht die Gruppe $-OR^4$ in Formel I bevorzugt der Formel $-NR^{12}R^{13}$, worin $R^{12}$ und $R^{13}$ unabhängig voneinander für H, $OCH_3$, $C_1$-$C_{12}$-, vorzugsweise $C_1$-$C_6$-Alkyl, Cyclopentyl, Cyclohexyl, Phenyl, Benzyl, oder $R^{12}$ und $R^{13}$ zusammen Tetra- oder Pentamethylen oder 3-Oxapentylen stehen. Beispiele für Alkyl sind zuvor angegeben worden.

Im Falle der Halogenide bedeutet die Gruppe $-OR^4$ in Formel I bevorzugt F, Br und insbesondere Cl.

Im Falle der Salze bedeutet $R^4$ z.B. ein Metall- oder Ammoniumkation. Bevorzugte Metallkationen sind Erdalkali- und besonders Alkalimetallkationen. Bevorzugte Ammoniumkationen sind $NH_4^{\oplus}$ und die Kationen von primären, sekundären und tertiären Aminen, die mit $C_1$-$C_6$-Alkyl, $C_1$-$C_6$-Hydroxyalkyl, Cyclopentyl, Cyclohexyl, Phenyloder Benzyl, Tetra- oder Pentamethylen oder 3-Oxapentylen substituiert sein können.

Das erfindungsgemässe Verfahren wird bevorzugt bei Temperaturen von 30 bis 150°C, besonders 50 bis 120°C, und insbesondere 50 bis 100°C, und zweckmässig in Gegenwart eines inerten, polaren und aprotischen Lösungsmittels durchgeführt. Geeignete Lösungsmittel sind z.B. Ether, wie z.B. Diethylether, Dibutylether, Ethylenglykoldimethylether, Diethylenglykoldimethylether, Tetrahydrofuran und Dioxan; Ester

wie z.B. Essigsäuremethylester; Nitrile wie z.B. Acetonitril; Sulfone und Sulfoxide wie z.B. Dimethylsulfon, Tetramethylensulfon und Dimethylsulfoxid; und halogenierte aromatische Kohlenwasserstoffe wie z.B. Chlorbenzol und Chlortoluol.

Die Reaktanden werden in mindestens molaren Verhältnissen eingesetzt. Es hat sich als vorteilhaft erwiesen, dass man Zn in einem Ueberschuss von bis zu 0,1 Mol und das Chlorsilan in einem Ueberschuss bis zu 0,5 Mol verwendet, bezogen auf die Verbindung der Formel II.

Das Verfahren kann so durchgeführt werden, dass man die Reaktanden und gegebenenfalls ein Lösungsmittel in einem Reaktionsgefäss vorlegt und danach erwärmt, bis die Reaktion beendet ist. Die Reaktion wird zweckmässig initiiert, z.B. durch Zugabe von wenig Jod. Das Zink kann zuvor aktiviert werden (vgl. Fieser u. Fieser, Reagents for Organic Synthesis, Vol. I, John Wiley, N.Y., S. 1276 (1967)). Das Zink wird vorteilhaft in Form von Zinkstaub eingesetzt.

Die Isolierung der Verbindungen der Formel I kann in üblicher Weise erfolgen, z.B. indem man das ausgefallene Zinkhalogenid abfiltriert, das verwendete Lösungsmittel abdestilliert und danach den Rückstand je nach Konsistenz durch Kristallisation, Destillation oder chromatographische Methoden weiter reinigt.

Die Carbonsäuren der Formel I werden zweckmässig hergestellt, indem man das Reaktionsgemisch gegebenenfalls nach Entfernen des Zinkhalogenids hydrolisiert, z.B. mit Wasser, und danach das mit z.B. NaOH basisch gestellte Hydrolysat mit einem Kohlenwasserstoff (Pentan, Hexan, Methylcyclohexan) extrahiert. Die wässrige Phase wird dann angesäuert, z.B. mit Salzsäure, und die saure wässrige Phase mit einem polaren aprotischen Lösungmittel extrahiert. Ein geeignetes Lösungmittel ist z.B. Essigsäureethylester. Die organische Phase wird in üblicher Weise getrocknet, z.B. mit $MgSO_4$, das Lösungsmittel entfernt und das Produkt z.B. mittels Destillation weiter gereinigt.

Die Säurederivate von Verbindungen der Formel I werden nach üblichen Methoden erhalten, z.B. durch Behandlung der Säuren mit wasserentziehenden Mitteln (z.B. Phosphorpentoxid) bzw. Halogenierungsmitteln (z.B. $PCl_5$) zur Herstellung der Anhydride bzw. Halogenide, sowie durch Veresterung, Umesterung, Amidierung oder Salzbildung zur Herstellung der Ester, Amide und Salze.

Ein weiterer Gegenstand der Erfindung sind die neuen Verbindungen der Formel II

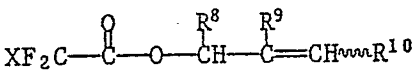 (II)

worin ∿ für Cis-/Transisomere steht, X für Cl oder Br steht, $R^8$ H, $C_1$-$C_{24}$-Alkyl oder gegebenenfalls in der Arylgruppe mit $C_1$-$C_6$-Alkoxy, $C_1$-$C_6$-Alkylthio, F, Cl, Br oder -CN substituiertes $C_6$-$C_{12}$-Aryl, $C_7$-$C_{16}$-Aralkyl oder $C_8$-$C_{30}$-Alkaralkyl darstellt, $R^9$ und $R^{10}$ unabhängig voneinander H oder $C_1$-$C_{24}$-Alkyl bedeuten, $R^8$ und $R^9$ oder $R^9$ und $R^{10}$ zusammen gegebenenfalls mit $C_1$-$C_6$-Alkyl-substituiertes $C_1$-$C_6$-Alkylen oder $C_3$-$C_6$-Alkenylen bedeuten, oder $R^8$ und $R^{10}$ zusammen gegebenenfalls mit $C_1$-$C_6$-Alkyl substituiertes $C_1$-$C_5$-Alkylen oder $C_2$-$C_5$-Alkenylen darstellen.

Die Bevorzugungen von $R^8$, $R^9$ und $R^{10}$ entsprechen den Bevorzugungen von $R^1$, $R^2$ bzw. $R^3$.

In einer besonderen Ausführungsform entsprechen in Formel II $R^8$ $C_1$-$C_{20}$-Alkyl und $R^9$ und $R^{10}$ H.

Eine weitere bevorzugte Ausführungsform sind Verbindungen der Formel II, worin $R^8$ für H, $C_1$-$C_{20}$-Alkyl und $R^9$ und $R^{10}$ für H oder $C_1$-$C_6$-Alkyl stehen.

Eine andere Ausführungsform sind Verbindungen der Formel II, worin $R^9$ H bedeutet und $R^8$ und $R^{10}$ zusammen gegebenenfalls mit $C_1$-$C_6$-Alkyl substituiertes $C_1$-$C_6$-Alkylen oder $C_2$-$C_5$-Alkenylen darstellen, oder $R^{10}$ für H steht und $R^8$ und $R^9$ zusammen gegebenenfalls mit $C_1$-$C_6$-Alkyl substituiertes $C_1$-$C_6$-Alkylen oder $C_3$-$C_6$-Alkenylen bedeuten, oder $R^8$ für H steht und $R^9$ und $R^{10}$ gegebenenfalls mit $C_1$-$C_6$-Alkyl substituiertes $C_1$-$C_6$-Alkylen oder $C_3$-$C_6$-Alkenylen darstellen.

Die Verbindungen der Formel II sind in an sich bekannter Weise durch Veresterung von Brom- bzw. Chlordifluoressigsäure mit Allylalkoholen der Formel III

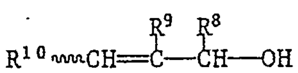 (III)

herstellbar. Die Allylalkohole der Formel III sind bekannt oder können in bekannter Weise durch die Umsetzung von Aldehyden der Formel $R^8$-CH=O mit Vinylmagnesiumbromiden der Formel

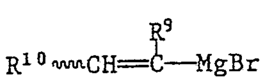

und anschliessende Hydrolyse hergestellt werden.

Ein weiterer Gegenstand vorliegender Erfindung sind Verbindungen der Formel Ia

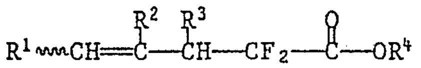 (Ia),

worin ~ für Cis-/Transisomere steht, $R^1$ H, $C_1$-$C_{24}$-Alkyl oder gegebenenfalls in der Arylgruppe mit $C_1$-$C_6$-Alkoxy, $C_1$-$C_6$-Alkylthio, F, 'Cl, Br oder -CN substituiertes $C_6$-$C_{12}$-Aryl, $C_7$-$C_{16}$-Aralkyl oder $C_8$-$C_{40}$-Alkaralkyl darstellt, $R^2$ und $R^3$ unabhängig voneinander H oder $C_1$-$C_{24}$-Alkyl bedeuten, $R^1$ und $R^3$ zusammen gegebenenfalls mit $C_1$-$C_6$-Alkyl substituiertes $C_1$-$C_5$-Alkylen oder $C_2$-$C_5$-Alkenylen darstellen, $R^2$ und $R^3$ zusammen oder $R^1$ und $R^2$ zusammen gegebenenfalls mit $C_1$-$C_6$-Alkyl substituiertes $C_1$-$C_6$-Alkylen oder $C_3$-$C_6$-Alkenylen bedeuten, und $R^4$ für $-SiR^5R^6R^7$ steht, worin $R^5$, $R^6$ und $R^7$ unabhängig voneinander $C_1$-$C_{12}$-Alkyl, Phenyl, oder Benzyl bedeuten, sowie deren Säuren und Säurederivate, mit der Massgabe, dass $R^1$, $R^2$ und $R^3$ gemeinsam nicht H bedeuten.

Die Bevorzugungen von $R^1$, $R^2$, $R^3$ und $R^4$ entsprechen den Bevorzugungen von $R^1$, $R^2$, $R^3$ und $R^4$ in den Verbindungen der Formel I.

In einer bevorzugten Ausführungsform ist in Formel Ia $R^1$ $C_1$-$C_{20}$-Alkyl und $R^2$ und $R^3$ H.

Eine andere bevorzugte Ausführungsform von Verbindungen der Formel Ia sind solche, worin $R^2$ H bedeutet und $R^1$ und $R^3$ zusammen gegebenenfalls mit $C_1$-$C_6$-Alkyl substituiertes $C_1$-$C_6$-Alkylen oder $C_2$-$C_5$-Alkenylen darstellen, oder $R^3$ für H steht und $R^1$ und $R^2$ zusammen gegebenenfalls mit $C_1$-$C_6$-Alkyl substituiertes $C_1$-$C_6$-Alkylen oder $C_3$-$C_6$-Alkenylen bedeuten, oder $R^1$ für H steht und $R^2$ und $R^3$ gegebenenfalls mit $C_1$-$C_6$-Alkyl substituiertes $C_1$-$C_6$-Alkylen oder $C_3$-$C_6$-Alkenylen darstellen.

Die Verbindungen der Formeln I und Ia und deren Derivate, besonders die Carbonsäuren, eignen sich als Monomere zur Herstellung von Homolymeren oder zusammen mit mono- oder diolefinischen Monomeren zur Herstellung von Copolymeren. Diese Polymeren können als Ionenaustauscherharze verwendet werden. Die Polymeren mit Carboxylgruppen können mit Uebergangsmetallen oder Uebergangsmetallverbindungen in metallorganische Polymere umgewandelt werden, deren Carboxylgruppen teilweise oder ganz mit den Uebergangsmetallen oder Uebergangsmetallverbindungen umgesetzt sind und die wertvolle Katalysatoren darstellen (vgl. DE-OS 2 605 247). Geeignete Comonomere sind z.B. Olefine (Ethylen, Propylen, Butylen, Styrol, Acryl- oder Methacrylsäure sowie deren Ester und Amide, Acrylnitril und Divinylbenzol).

Die nachfolgenden Beispiele erläutern die Erfindung näher.

Beispiel : Chlordifluoressigsäure-allylester

In einem 1,5 1 Sulfierkolben mit Wasserabscheider werden 294,0 g (2.25 Mol) Chlordifluoressigsäure (purum, Fa. Fluka AG) und 228,0 g (3,93 Mol) Allylalkohol in 1 1 Chloroform gelöst, in Gegenwart von 11,25 g p-Toluolsulfonsäure (0.06 mol) während 17 Stunden am Rückfluss gehalten, wobei sich 41 ml $H_2O$ abscheiden. Die gelbe Reaktionslösung wird 2x mit $H_2O$ und 3x mit gesättiger wässriger $NaHCO_3$-Lösung gewaschen, über $MgSO_4$ getrocknet und über eine Vigreux-Kolonne bei Normaldruck destilliert. Ausbeute: 306,0 g (80 %) farbloses Oel vom Siedepunkt 98-100°C/988 mbar.

Beispiele 2-8:

Analog Beispiel 1 werden mit substituierten Allylalkoholen weitere Chlordifluoressigsäure-allylester hergestellt. Die zugehörigen Daten sind in Tabelle 1 zusammengefasst.

Tabelle 1

| Beispiel No. | $ClF_2C-CO-R$ R = | Siedepunkt [°C/mbar] | $^1H$-NMR ($CDCl_3$) $\delta(-O-C\underline{H}_n-R)$ |
|---|---|---|---|
| 1 | $OCH_2CH=CH_2$ | 98-100/988 | 4,82 ppm, 2H |
| 2 | $OCH_2C(CH_3)=CH_2$ | 110/988 | 4,92 ppm, 2H |
| 3 | $OCH_2CH=CHCH_3$ | 52/32,5 | 4,78 ppm, 2H |
| 4 | $OCHCH=CH_2$ $(CH_2)_4CH_3$ | Oel a) | ca. 5,3 ppm, 1H |
| 5 | $OCH_2CH=CH(CH_2)_2CH_3$ | Oel a) | 4,80 ppm, 2H |
| 6 | $OCHCH=CHCH_3$ $CH_2CH_3$ | Oel a) | 5,26 ppm, 1H |
| 7 | $OCH_2CH=CHC_6H_5$ | Oel a) | 5,00 ppm, 2H |
| 8 | 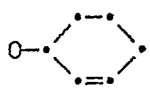 | Oel a) | 5,43 ppm, 1H |

a) Reinigung durch Chromatographie an Kieselgel mit $CH_2Cl_2$.

Beispiel 9: 2,2-Difluor-4-pentensäure

In einem Glasautoklav werden 100,0 g (0,59 Mol) Chlordifluoressigsäure-allylester, 42,2 g (0,65 Mol) Zinkstaub (aktiviert nach Fieser & Fieser), 96,7 g (0,88 Mol) Trimethylchlorsilan und 400 ml absolutes Acetonitril zusammengegeben, mit einer Spatelspitze Jod versetzt und während 48 Stunden auf 100°C erhitzt. Vom ausgefallenen Zinksalz wird abfiltriert, mit $H_2O$ hydrolsiert, und mit 2N NaOH basisch gestellt. Die mit Pentan extrahierte dunkelbraune wässrige Phase wird mit halbkonzentrierter HCl angesäuert und das Produkt mit Essigsäureethylester extrahiert. Nach dem Trocknen der organischen Phase über $MgSO_4$ und Eindampfen am Rotationsverdampfer (RV) wird der Rückstand am Vakuum destilliert. Ausbeute: 59,4 g (74 %) farbloses Oel vom Siedepunkt 73-75°C/15,6 mbar.

Beispiele 10-15:

Analog Beispiel 9 werden die Chlordifluoressigsäure-allylester der Beispiele 2-6 und 8 umgesetzt. Die zugehörigen Daten sind in Tabelle 2 angegeben.

Tabelle 2

| Beispiel No. | Edukt aus Beispiel No. | Produkt | $^1H$-NMR ($CDCl_3$) $\delta(-CH_n-CF_2-COOR)$ |
|---|---|---|---|
| 9 | 1 | $CH_2=CHCH_2CF_2COOH$ | 2,87 ppm, 2H |
| 10 | 2 | $CH_2=C(CH_3)CH_2CF_2COOH$ | 2,83 ppm, 2H |
| 11 | 3 | $CH_2=CHCH(CH_3)CF_2COOH$ | 2,95 ppm, 1H |
| 12 | 4 | $CH_3(CH_2)_4CH=CHCH_2CF_2COOH$ | 2,81 ppm, 2H |
| 13 | 5 | 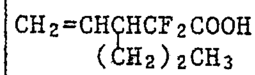 | 2,78 ppm, 1H |
| 14 | 6 | $CH_3CH_2CH=CHCH(CH_3)CF_2COOH$ | 2,91 ppm, 1H |
| 15 | 8 | 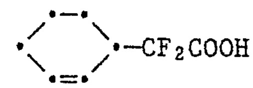 | 2,95 ppm, 1H |

Beispiel 16: 2,2-Difluor-4-pentensäure-Natriumsalz

In 20 ml Methanol werden 4,01 g (29,9 mMol) 2,2-Difluor-4-pentensäure gelöst und bei Raumtemperatur (RT) mit 1 Aequivalent $NaOCH_3$ in Methanol versetzt. Nach dem Eindampfen am RV verbleiben 4,65 g (100 %) farblose Kristalle vom Schmelzpunkt 168-170° C (Zersetzung).

Beispiel 17: 2,2-Difluor-4-pentensäurechlorid

In einem Rundkolben mit Destillationsaufsatz werden 57,2 g (0,42 Mol) 2,2-Difluor-4-pentensäure langsam zu 102,0 g Phosphorpentachlorid getropft. Nach 20 Minuten Rühren bei RT wird das Reaktionsgemisch destilliert, wobei 42,1 g (65 %) Säurechlorid als farbloses Oel bei 92-98° C/988 mbar übergeht. $^1H$-NMR (60 MHz, $CDCl_3$): 6,0-5,0 (m, 3H); 2,9 (d x t, J = 6; 16 Hz, 2H).

Beispiel 18: 2,2-Difluor-4-pentensäure-ethylester

Analog Beispiel 1 werden 38,5 g (0,28 Mol) 2,2-Difluor-4-pentensäure mit Ethanol verestert. Ausbeute: 41,3 g (89 %) farbloses Oel vom Siedepunkt 55-57° C/52 mbar, $^1H$-NMR (60 MHz, $CDC1_3$): 6,1-5,0 (m, 3H); 4,30 (qa, J = 7 Hz, 2H); 2,85 (d x t, J = 6; 16 Hz, 2H); 1,35 (t, J = 7 Hz, 3H).

Beispiel 19: 2,2-Difluor-4-pentensäure-amid

Die Aminolyse von 30,0 g (0,18 Mol) 2,2-Difluor-4-pentensäure-ethylester (vgl. Beispiel 18) mit 45 ml 25 %-igem wässrigem Ammoniak bei RT ergibt nach Extraktion mit Essigsäureethylester und Destillation (130° C/26 mbar) 22,3 g (91 %) farbloses kristallines Amid. $^1H$-NMR (300 MHz, $CDCl_3$): 6,4 (breit, $NH_2$); 5,85-5,69 (m, 1H); 5,35 -5,24 (m, 2H); 2,86 (m, 2H).

**Patentansprüche**

1. Verfahren zur Herstellung von Verbindungen der Formel I

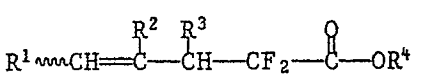 (I)

worin ∼ für Cis-/Transisomere steht, $R^1$ H, $C_1$-$C_{24}$-Alkyl oder gegebenenfalls in der Arylgruppe mit $C_1$-$C_6$-Alkoxy, $C_1$-$C_6$-Alkylthio, F, Cl, Br oder -CN substituiertes $C_6$-$C_{12}$-Aryl, $C_7$-$C_{16}$-Aralkyl oder $C_8$-$C_{40}$-Alkaralkyl darstellt, $R^2$ und $R^3$ unabhängig voneinander H oder $C_1$-$C_{24}$-Alkyl bedeuten, $R^1$ und $R^3$ zusammen gegebenenfalls mit $C_1$-$C_6$-Alkyl substituiertes $C_1$-$C_5$-Alkylen oder $C_2$-$C_5$-Alkenylen darstellen, $R^2$ und $R^3$ zusammen oder $R^1$ und $R^2$ zusammen gegebenenfalls mit $C_1$-$C_6$-Alkyl substituiertes $C_1$-$C_6$-Alkyl substituiertes $C_1$-$C_6$-Alkenylen bedeuten, und $R^4$ für $-SiR^5R^6R^7$ steht, worin $R^5$, $R^6$ und $R^7$ unabhängig voneinander $C_1$-$C_{12}$-Alkyl, Phenyl oder Benzyl bedeuten, sowie deren Säuren und

Säurederivate, dadurch gekennzeichnet, dass man 1 Mol eines Monobrom- oder Monochlordifluoressigsäureallylesters der Formel II

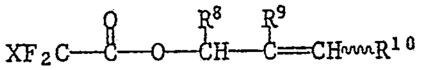 (II),

worin ∼ die zuvor angegebene Bedeutung hat, X für Cl oder Br steht, $R^8$ die Bedeutungen von $R^1$, $R^9$ die Bedeutungen von $R^2$, $R^{10}$ die Bedeutungen von $R^3$, $R^8$ und $R^9$ die Bedeutungen von $R^1$ und $R^2$, $R^8$ und $R^{10}$ die Bedeutungen von $R^1$ und $R^3$ und $R^9$ und $R^{10}$ die Bedeutungen von $R^2$ und $R^3$ haben, in Gegenwart von mindestens 1 Mol Zink und von mindestens 1 Mol eines Monochlorsilans $R^5R^6R^7SiCl$, worin $R^5$, $R^6$ und $R^7$ die zuvor angegebene Bedeutung haben, erwärmt, die Verbindungen der Formel I isoliert oder zur Herstellung der Säuren hydrolisiert und gegebenenfalls aus den Säuren deren Säurederivate herstellt.

2. Verfahren gemäss Anspruch 1, dadurch gekennzeichnet, dass in Formel I $R^1$ H, $C_1$-$C_{20}$-Alkyl, gegebenenfalls in der Phenylgruppe mit $C_1$-$C_6$-Alkoxy, $C_1$-$C_6$-Alkylthio, F, Cl, Br oder -CN substituiertes Phenyl, $C_7$-$C_{16}$-Phenylalkyl oder $C_8$-$C_{30}$-Alkylphenylalkyl bedeutet, $R^2$ und $R^3$ unabhängig voneinander H oder $C_1$-$C_{12}$-Alkyl, $R^1$ und $R^3$ zusammen $C_1$-$C_3$-Alkylen oder $C_2$-$C_3$-Alkenylen darstellen, $R^2$ und $R^3$ zusammen oder $R^1$ und $R^2$ zusammen $C_1$-$C_4$-Alkylen, $R^2$ und $R^3$ zusammen oder $R^1$ und $R^2$ zusammen $C_3$-$C_4$-Alkenylen sind.

3. Verfahren gemäss Anspruch 1, dadurch gekennzeichnet, dass $R^1$ für H, $C_1$-$C_{20}$-Alkyl und $R^2$ und $R^3$ für H oder $C_1$-$C_6$-Alkyl stehen.

4. Verfahren gemäss Anspruch 1, dadurch gekennzeichnet, dass $R^5$, $R^6$ und $R^7$ für Methyl stehen.

5. Verfahren gemäss Anspruch 1, dadurch gekennzeichnet, dass es sich bei den Säurederivaten um Anhydride, Ester, Amide, Halogenide oder Salze der Carbonsäuren der Formel I, worin $R^4$ H ist, handelt.

6. Verfahren gemäss Anspruch 1, dadurch gekennzeichnet, dass es bei einer Temperatur von 30 bis 150°C durchgeführt wird.

7. Verfahren gemäss Anspruch 1, dadurch gekennzeichnet, dass es in Gegenwart eines inerten polaren, aprotischen Lösungsmittels durchgeführt wird.

8.Verbindungen der Formel Ia

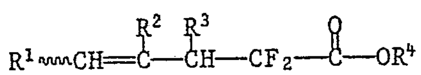 (Ia),

worin ∼ für Cis-/Transisomere steht, $R^1$ H, $C_1$-$C_{24}$-Alkyl oder gegebenenfalls in der Arylgruppe mit $C_1$-$C_6$-Alkoxy, $C_1$-$C_6$-Alkylthio, F, Cl, Br oder -CN substituiertes $C_6$-$C_{12}$-Aryl, $C_7$-$C_{16}$-Aralkyl oder $C_8$-$C_{40}$-Alkaralkyl darstellt, $R^2$ und $R^3$ unabhängig voneinander H oder $C_1$-$C_{24}$-Alkyl bedeuten, $R^1$ und $R^3$ zusammen gegebenenfalls mit $C_1$-$C_6$-Alkyl substituiertes $C_1$-$C_5$-Alkylen oder $C_2$-$C_5$-Alkenylen darstellen, $R^2$ und $R^3$ zusammen oder $R^1$ und $R^2$ zusammen gegebenenfalls mit $C_1$-$C_6$-Alkyl substituiertes $C_1$-$C_6$-Alkylen oder $C_3$-$C_6$-Alkenylen bedeuten, und $R^4$ für $-SiR^5R^6R^7$ steht, worin $R^5$, $R^6$ und $R^7$ unabhängig voneinander $C_1$-$C_{12}$-Alkyl, Phenyl, oder Benzyl bedeuten, sowie deren Säuren und Säurederivate, mit der Massgabe, dass $R^1$, $R^2$ und $R^3$ gemeinsam nicht H bedeuten.

9. Verbindungen gemäss Anspruch 8, worin in Formel Ia $R^1$ H, $C_1$-$C_{20}$-Alkyl, gegebenenfalls in der Phenylgruppe mit $C_1$-$C_6$-Alkoxy, $C_1$-$C_6$-Alkythio, F, Cl, Br oder -CN substituiertes Phenyl, $C_7$-$C_{16}$-Phenylalkyl oder $C_8$-$C_{30}$-Alkylphenylalkyl bedeutet, $R^2$ und $R^3$ unabhängig voneinander H oder $C_1$-$C_{12}$-Alkyl, $R^1$ und $R^3$ zusammen $C_1$-$C_3$-Alkylen oder $C_2$-$C_3$-Alkenylen darstellen, $R^2$ und $R^3$ zusammen oder $R^1$ und $R^2$ zusammen $C_1$-$C_4$-Alkylen oder $C_3$-$C_4$-Alkenylen sind.

10. Verbindungen gemäss Anspruch 8, worin in Formel Ia $R^1$ für H, $C_1$-$C_{20}$-Alkyl und $R^2$ und $R^3$ für H oder $C_1$-$C_6$-Alkyl stehen.

11. Verbindungen gemäss Anspruch 10, worin $R^1$ für $C_1$-$C_{20}$-Alkyl und $R^2$ und $R^3$ für H stehen.

12. Verbindungen gemäss Anspruch 8, worin in Formel Ia $R^2$ H bedeutet und $R^1$ und $R^3$ zusammen gegebenenfalls mit $C_1$-$C_6$-Alkyl substituiertes $C_1$-$C_6$-Alkylen oder $C_2$-$C_5$-Alkenylen darstellen, oder $R^3$ für H steht und $R^1$ und $R^2$ zusammen gegebenenfalls mit $C_1$-$C_6$-Alkyl substituiertes $C_1$-$C_6$-Alkylen oder $C_3$-$C_6$-Alkenylen bedeuten, oder $R^1$ für H steht und $R^2$ und $R^3$ gegebenenfalls mit $C_1$-$C_6$-Alkyl substituiertes $C_1$-$C_6$-Alkylen oder $C_3$-$C_6$-Alkenylen darstellen.

13. Verbindungen gemäss Anspruch 8, worin $R^4$ in Formel Ia $(CH_3)_3Si$bedeutet.

14. Verbindungen gemäss Anspruch 8, dadurch gekennzeichnet, dass es sich bei den Säurederivaten um Anhydride, Ester, Amide, Halogenide oder Salze der Carbonsäuren der Formel Ia mit $R^4$ = H handelt.

15. Verbindungen gemäss Anspruch 8, dadurch gekennzeichnet, dass für die Säuren und Säurederivate $R^4$ in Formel Ia H, $C_1$-$C_{18}$-Acyl, der um eine Hydroxylgruppe verminderte Rest eines einwertigen Alkohols, ein Metall- oder Ammoniumkation, oder die Gruppe $R^4O$- für F, Cl, Br, $-NH_2$ oder den um ein H-Atom verminderten Rest eines primären oder sekundären Amins steht.

16. Verbindungen der Formel II

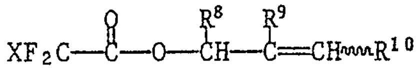 (II),

woring ⁓ für Cis-/Transisomere steht, X für Cl oder Br steht, $R^8$ H, $C_1$-$C_{24}$-Alkyl oder gegebenenfalls in der Arylgruppe mit $C_1$-$C_6$-Alkoxy, $C_1$-$C_6$-Alkylthio, F, Cl, Br oder -CN substituiertes $C_6$-$C_{12}$-Aryl, $C_7$-$C_{16}$-Aralkyl oder $C_8$-$C_{30}$-Alkaralkyl darstellt, $R^9$ und $R^{10}$ unabhängig voneinander H oder $C_1$-$C_{24}$-Alkyl bedeuten, $R^8$ un $R^9$ oder $R^9$ und $R^{10}$ zusammen gegebenenfalls mit $C_1$-$C_6$-Alkyl-substituiertes $C_1$-$C_6$-Alkylen oder $C_3$-$C_6$-Alkenylen bedeuten, oder $R^8$ und $R^{10}$ zusammen gegebenenfalls mit $C_1$-$C_6$-Alkyl substituiertes $C_1$-$C_5$-Alkylen oder $C_2$-$C_5$-Alkenylen darstellen.

17. Verbindungen gemäss Anspruch 16, worin in Formel II $R^8$ H, $C_1$-$C_{20}$-Alkyl, gegebenenfalls in der Phenylgruppe mit $C_1$-$C_6$-Alkoxy, $C_1$-$C_6$-Alkylthio, F, Cl, Br oder -CN substituiertem Phenyl, $C_7$-$C_{16}$-Phenylalkyl oder $C_8$-$C_{30}$-Alkylphenylalkyl bedeutet, $R^9$ und $R^{10}$ unabhängig voneinander H oder $C_1$-$C_{12}$-Alkyl, $R^8$ und $R^{10}$ zusammen $C_1$-$C_3$-Alkylen oder $C_2$-$C_3$-Alkenylen darstellen, $R^9$ und $R^{10}$ zusammen oder $R^8$ und $R^9$ zusammen $C_1$-$C_4$-Alkylen, $R^9$ und $R^{10}$ zusammen $C_3$-$C_4$-Alkenylen oder $R^8$ und $R^9$ zusammen $C_3$-$C_4$-Alkenylen sind.

18. Verbindungen gemäss Anspruch 16, worin in Formel II $R^8$ für H, $C_1$-$C_{20}$-Alkyl und $R^9$ und $R^{10}$ für H oder $C_1$-$C_6$-Alkyl stehen.

19. Verbindungen gemäss Anspruch 18, worin $R^8$ für $C_1$-$C_{20}$-Alkyl und $R^9$ und $R^{10}$ für H stehen.

20. Verbindungen gemäss Anspruch 16, worin in Formel II $R^9$ H bedeutet und $R^8$ und $R^{10}$ zusammen gegebenenfalls mit $C_1$-$C_6$-Alkyl substituiertes $C_1$-$C_6$-Alkylen oder $C_2$-$C_5$-Alkenylen darstellen, oder $R^{10}$ für H steht und $R^8$ und $R^9$ zusammen gegebenenfalls mit $C_1$-$C_6$-Alkyl substituiertes $C_1$-$C_6$-Alkylen oder $C_3$-$C_6$-Alkenylen bedeuten, oder $R^8$ für H steht und $R^9$ und $R^{10}$ zusammen gegebenenfalls mit $C_1$-$C_6$-Alkyl substituiertes $C_1$-$C_6$-Alkylen oder $C_3$-$C_6$-Alkenylen darstellen.